# EUROPEAN PATENT APPLICATION

(11) **EP 3 742 174 A1**
(43) Date of publication of application: **25.11.2020**
(21) Application number: 19774043.4
(22) Date of filing: 04.04.2019
(51) Int. Cl.: G01N 35/08

(54) **MULTI-SYSTEM FOR SIMULTANEOUSLY PERFORMING BIOCHEMICAL TEST AND BLOOD TEST, AND MULTI-DISC USED THEREFOR**

(71) Applicant: Biobank Inc., Okcheon-gun, Chungcheongbuk-do 29046 (KR)
(72) Inventor: PARK, Sung Sik, Seo-gu, Daejon 35389 (KR)
(74) Representative: Susanetto, Carlo
(86) International application number: PCT/KR2019/004014
(87) International publication number: WO 2020/204232

(57) **Abstract**

The present invention relates to a multisystem for simultaneously performing a biochemical examination and a blood test or an immunity test, the multisystem including a multi-disc 1 in which a biochemical examination unit 100, a blood testing unit 200 and an immunity test unit 300 are divided and formed in a fan shape based on a rotation center. There is an effect that it is possible to perform all of a biochemical examination for measuring concentration values of biochemical components of serum to diagnose vital functions, a blood testing for separating blood samples to measure and test the number and deformation of red blood cells or white blood cells, and an immunity test for measuring an antigen-antibody reaction of serum to diagnose and test cancer or viruses in one device at the same time by using the multi-disc 1 according to the present invention.

## Description

### Field of the invention

The present invention relates to a multisystem for simultaneously performing a biochemical examination and a blood test or an immunity test in one platform and, more specifically, to a multisystem for simultaneously performing the biochemical examination and the blood test or the immunity test, the multisystem which is constructed such that the multisystem can rapidly and easily evaluate whether a reaction material is reacted or not with respect to blood components using a disc based on centrifugal force, and can measure and inspect in one platform all of a biochemical measurement test using an enzyme reaction in serum of blood, the blood test of counting the number of red blood cells or white blood cells using capillary method, dyeing method or the like, and the immunity test of diagnosing and testing cancer or virus by measuring an antigen-antibody reaction of the serum, and a multi-disc used in the multisystem.

### Background of the invention

Since blood not only performs an important role of supplying oxygen, nutrients, hormone and nutritive substances to cells of all organs within the body, but also performs a function of transporting toxin and waste as a protective organ against infection, changes in the body may change the blood components. Therefore, respective components of blood should be tested to accurately determine diagnosis, treatment and prognosis of diseases.

In order to perform the above-mentioned blood test, special chemical test along with morphological, biochemical and immune serum examinations of a collected blood are carried out.

Here, the morphological examination of the blood diagnoses bleeding tendency, hematodyscrasia, infectious disease, etc., and determines progress and prognosis of various diseases by conducting a blood cell examination of measuring hemoglobin, hematocrit (hematocrit volume), red blood cells, white blood cells, platelet count, cell percentages or the white blood cells, etc. using whole blood, conducting a blood smear test of thinly applying blood to a slide and dyeing the blood thinly applied to the slide to directly observe shape, number or the like of blood cells with a microscope, and inspecting bleeding time, coagulation time, capillary vessel resistance, etc. At this time, measurement processes are performed using an electrochemical method, a dyeing method, etc.

Further, a biochemical method or an immunological method, and a special chemical method comprise first removing blood cells within the blood using centrifugation or the like and carrying out a testing operation using a supernatant (serum or plasma).

In general, the biochemical method comprises selecting a method of mainly reacting serum (or plasma) with each of indicators to obtain a reaction product, measuring an absorbance value with a specific wavelength of the reaction product, and converting the measured absorbance value into a concentration value, and the biochemical method is divided into a wet type biochemical method and a dry type biochemical method.

The wet type biochemical method, as a mostly selected method, enables a reaction with serum (or plasma) to occur since an indicator is formed in the form of liquid. In order to perform a measurement process more simply, the dry type biochemical method comprises an absorbance value by solidifying the indicator, coating a solidified indicator on a film, and then reacting the solidified indicator coated on the film with serum (or plasma).

Further, the immune serum examination, as an examination of antigens and antibodies for various bacteria and viruses, determines the cause of allergies by diagnosing hepatitis B, hepatitis C, acquired immune deficiency syndrome (AIDS), syphilis, etc., diagnosing rheumatism disease, separating various immunocytes from blood to perform a process of examining function of the immunocytes, and measuring a specific immunoglobulin within the blood for material that becomes various causes of other allergies.

Most cases of the immune serum examination adopt a method of measuring an amount of the antibody (antigen) by labelling a marker on an antibody (antigen) to be generally examined using an antigen-antibody immune reaction.

A lot of technical developments have recently been made to simultaneously perform large quantities of laboratory test along with such examinations using blood, and examples of the technical developments include methods of DNA chip, lab-on-a-chip, etc. These methods are mainly a method for carrying out the immune serum examination and genetic testing at the same time.

In order to have the same way of doing the technical developments, it is an urgently demanded circumstance to develop examination devices and kits capable of conducting all of the morphological examination for counting the number of blood cells, the biochemical examination, the immune serum examination, and the genetic testing in one device at the same time.

### (Prior art documents)

(Patent document 1) Korean Patent Registration No. 10-0748181
(Patent document 2) Korean Patent Registration No. 10-1608598

### SUMMARY OF THE INVENTION

The present invention has been devised to solve the aforementioned problem, the purpose of the present invention is to provide a multisystem which is constructed such that the multisystem can rapidly and easily evaluate whether a reaction material is reacted or not with respect to blood components using a disc based on centrifugal force, and can simultaneously perform all of a biochemical examination, a blood test and an immunity test in one platform.

In order to solve the aforementioned problem, a multisystem for simultaneously performing a biochemical examination, a blood test and an immunity test includes
a multi-disc 1 in which a biochemical examination unit 100 and
a blood testing unit 200 are divided and formed in a fan shape based on a rotation center.

Further, in a multisystem according to the present invention,
the multi-disc 1
additionally, includes an immunity test unit 300 to perform immunity test at the same time.

Further, in a multisystem according to the present invention,
the multi-disc 1 includes:
an upper cover 10; and
a reaction chamber plate 20 which is detachably combined with or integrally formed with the upper cover 10.

Further, in a multisystem according to the present invention,
the upper cover 10 of the multi-disc 1 includes a first blood injection port 11 and a second blood injection port 12 which are provided at positions adjacent to the rotation center, and a plurality of reagent injection ports 13 which are formed to be distributed in an arc shape at the edge side.

The reaction chamber plate 20 includes a biochemical examination unit 100 for measuring concentration values of biochemical components of serum to diagnose vital functions, a blood testing unit 200 for separating blood samples to measure and test the number and deformation of red blood cells or white blood cells, and an immunity test unit 300 for measuring an antigen-antibody reaction of serum to diagnose and test cancer or viruses.

Further, in a multisystem according to the present invention,
the biochemical examination unit 100 of the multi-disc 1 includes:
a blood receipt chamber 110; a blood filter chamber 120 which is provided to be communicated with the blood receipt chamber 110; a plurality of serum distribution chambers 130 which are provided to be communicated with the blood filter chamber 120 through an arc-shaped distribution flow path; and a plurality of reagent reaction chambers 140 which are provided to be communicated with the plurality of serum distribution chambers 130.

Further, in a multisystem according to the present invention,
the blood receipt chamber 110 is formed in an arc shape around a rotation axis at a position adjacent to the rotation center to receive a blood sample supplied,
the blood filter chamber 120 is formed in an arc shape around the rotation axis of the rotation center and has a filter provided therein to filter a blood sample transferred,
in the plurality of serum distribution chambers 130, an inlet of each of the serum distribution chambers 130 is opened to the arc-shaped distribution flow path such that a predetermined amount of filtered blood components is distributed to each of the serum distribution chambers 130, and the inlet of each of the serum distribution chambers 130 is communicated with each of the corresponding reagent reaction chambers 140 through the flow path,

in the plurality of reagent reaction chambers 140, a plurality of chambers are formed to be distributed in the edge side, and the plurality of chambers are provided to cause a reaction between a reagent and the blood components supplied from the serum distribution chambers 130.

Further, in a multisystem according to the present invention,
a flow path connecting the blood receipt chamber 110 to the blood filter chamber 120 is formed in an outermost line from the rotation center of each chamber to facilitate transfer of blood by rotational centrifugal force.

Further, in a multisystem according to the present invention,
A connection flow path of the serum distribution chambers 130 and the reagent reaction chambers 140 is formed to form a predetermined angle with respect to a rotational direction to facilitate transfer of blood by rotational centrifugal force.

Further, in a multisystem according to the present invention,
the blood testing unit 200 includes:
a capillary tube holding chamber 210 which holds tubes containing blood samples; and
a dyeing chamber 220 which is provided to discriminate and count the white blood cells by treating the blood samples with a dyeing reagent, thereby dyeing five types of white blood cells.

Further, in a multisystem according to the present invention,
the immunity test unit 300 includes
a structure which centrifuges a blood sample injected through a separate injection port, and to which only a separated serum is supplied, or a structure which is communicated with the distribution flow path provided in the biochemical examination unit 100 to receive serum only, i.e., filtered blood components.

Further, a multisystem according to the present invention additionally includes:
a lighting unit 2 which irradiates light onto the multi-disc 1;
a camera unit 3 which photographs the multi-disc 1;
a display unit 4 which displays data received from the camera unit 3; and a DB unit 5.

Further, in a multisystem according to the present invention,
the lighting unit 2 is comprised of a light source 2a and a light filter 2b to irradiate a monochromatic light onto the multi-disc 1,
the camera unit 3 is disposed above the multi-disc 1 to transmit the measured absorbance data or captured image data to the display unit 4 by measuring absorbance values after a reaction of the multi-disc 1 or capturing images,
the display unit 4 displays the absorbance data and image data received from the camera unit 3, and
the DB unit 5 is interlocked with the camera unit 3 and the display unit 4, stores the measured data, and stores data compared with stored reference data.

Furthermore, a multi-disc according to the present invention includes:
a biochemical examination unit 100 which measures concentration values of biochemical components of serum to diagnose vital functions;
a blood testing unit 200 which separates blood samples to measure and test the number and deformation of red blood cells or white blood cells; and
an immunity test unit 300 which measures an antigen-antibody reaction of serum to diagnose and test cancer or viruses, wherein the multi-disc is formed by dividedly forming the biochemical examination unit 100, blood testing unit 200 and immunity test unit 300 in a fan shape based on the rotation center.

Further, the multi-disc according to the present invention includes:
an upper cover 10 including a first blood injection port 11 and a second blood injection port 12 which are provided at positions adjacent to the rotation center, and a plurality of reagent injection ports 13 which are formed to be distributed in an arc shape at the edge side; and
a reaction chamber plate 20 which is coupled to the upper cover 10, and includes a biochemical examination unit 100 for measuring concentration values of biochemical components of serum to diagnose vital functions, a blood testing unit 200 for separating blood samples to measure and test the number and deformation of red blood cells or white blood cells, and an immunity test unit 300 for measuring an antigen-antibody reaction of serum to diagnose and test cancer or viruses.

Further, in a multi-disc according to the present invention,
the biochemical examination unit 100 of the multi-disc includes:
a blood receipt chamber 110 which is provided at a position corresponding to the first blood injection port 11; a blood filter chamber 120 which is provided to be communicated with the blood receipt chamber 110; a plurality of serum distribution chambers 130 which are provided to be communicated with the blood filter chamber 120 through an arc-shaped distribution flow path; and a plurality of reagent reaction chambers 140 which are provided to be communicated with the plurality of serum distribution chambers 130 and provided at positions corresponding to the reagent injection ports 13,
the blood testing unit 200 of the multi-disc includes:
   a capillary tube holding chamber 210 which holds tubes containing blood samples; and a dyeing chamber 220 which is provided to discriminate and count the white blood cells by treating the blood samples with a dyeing reagent, thereby dyeing five types of white blood cells,
   the immunity test unit 300 of the multi-disc includes
   a structure which centrifuges a blood sample injected through a separate injection port, and to which only a separated serum is supplied, or a structure which is communicated with the distribution flow path provided in the biochemical examination unit 100 to receive serum only, i.e., filtered blood components.

When a multisystem for simultaneously performing a biochemical examination, a blood test and an immunity test according to the present invention, and a multi-disc used in the same are used, there is an effect that it is possible to perform all of a biochemical examination for measuring concentration values of biochemical components of serum to diagnose vital functions, a blood testing for separating blood samples to measure and test the number and deformation of red blood cells or white blood cells, and an immunity test for measuring an antigen-antibody reaction of serum to diagnose and test cancer or viruses in one device at the same time.

### BRIEF DESCRIPTION OF THE DRAWINGS

FIG. 1 is a drawing schematically illustrating the overall configuration of a multisystem for performing a biochemical examination, a blood test and an immunity test according to an embodiment of the present invention.
FIG. 2 is an enlarged view illustrating a multi-disc of FIG. 1.
FIG. 3 is an exploded view of FIG. 2.
FIG. 4 is a drawing illustrating a multi-disc configured to perform a biochemical examination and a blood test only according to other embodiment of the present invention.
FIG. 5 is a drawing schematically illustrating a combination relation of a multi-disc used in the multisystem with a multisystem for performing a biochemical examination, a blood test and an immunity test according to an embodiment of the present invention.

### DETAILED DESCRIPTION OF THE PREFERRED EMBODIMENT

Hereinafter, preferred embodiments of a multisystem for simultaneously performing a biochemical examination and a blood test that is the present invention will be described in more detail with reference to the accompanying drawings.

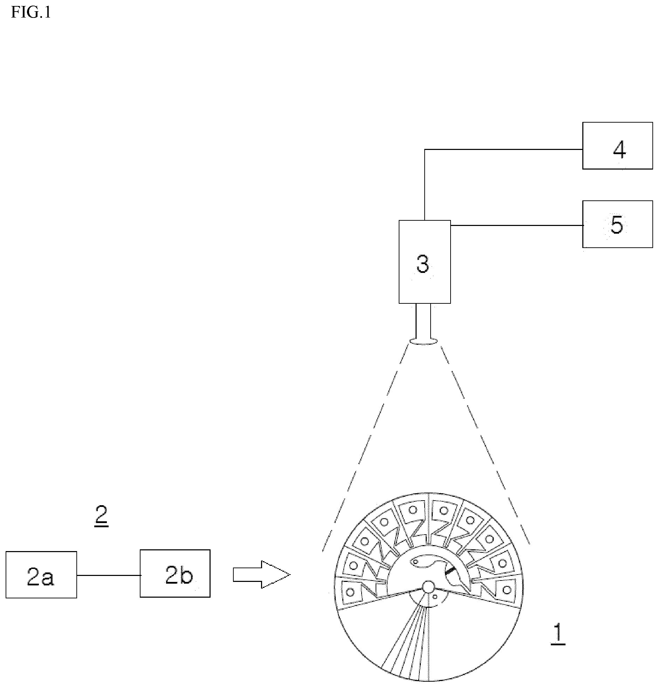

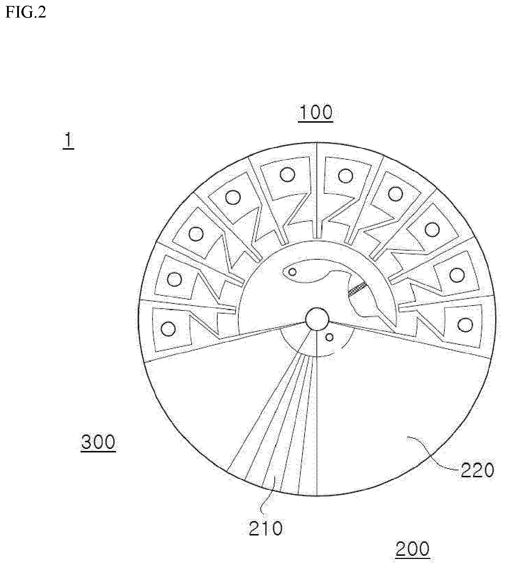

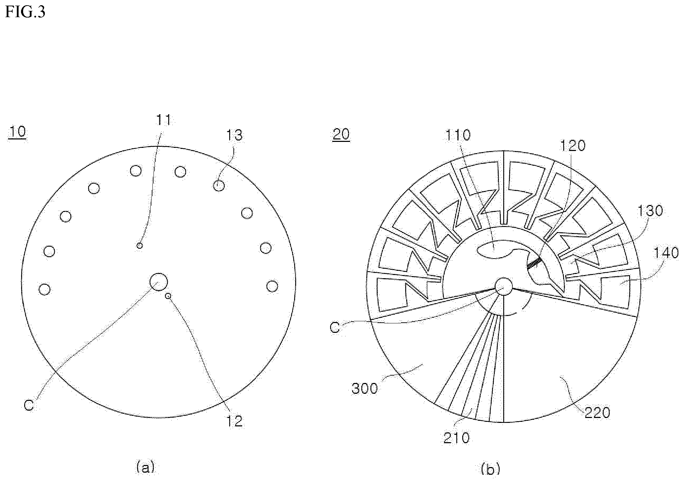

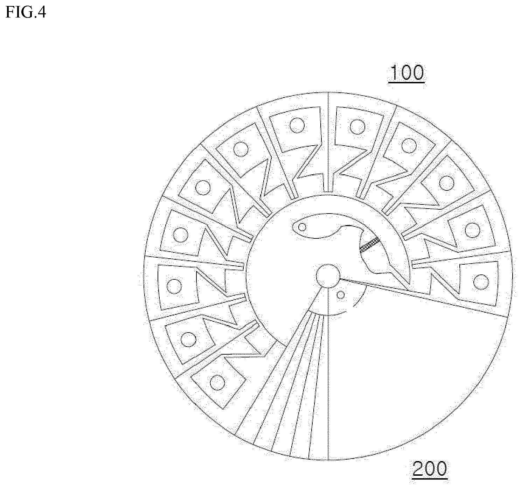

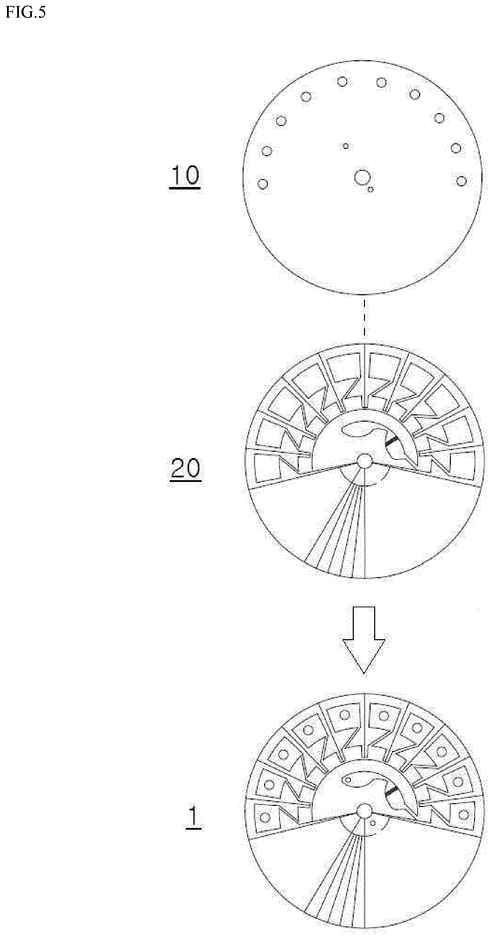

FIG. 1 is a drawing schematically illustrating the overall configuration of a multisystem for performing a biochemical examination, a blood test and an immunity test according to an embodiment of the present invention, FIG. 2 is an enlarged view illustrating a multi-disc of FIG. 1, FIG. 3 is an exploded view of FIG. 2, FIG. 4 is a drawing illustrating a multi-disc configured to perform a biochemical examination and a blood test only according to other embodiment of the present invention, and FIG. 5 is a drawing schematically illustrating a combination relation of a multi-disc used in the multisystem with a multisystem for performing a biochemical examination, a blood test and an immunity test according to an embodiment of the present invention.

As illustrated in FIG. 1 to FIG. 5, a multisystem for simultaneously performing a biochemical examination, a blood test and/or an immunity test according to the present invention includes a multi-disc 1, a lighting unit 2, a camera unit 3, a display unit 4, and a DB unit 5.

Here, the multi-disc 1 is constructed by dividedly forming the biochemical examination unit 100, blood testing unit 200 or immunity test unit 300 in a fan shape based on the rotation center, is rotated by a rotation control means (not illustrated) based on the rotation center C, and includes an upper cover 10 and a reaction chamber plate 20.

The upper cover 10 which is formed in the form of a disc type plate having a rotation center C may be detachably coupled to an upper portion of the reaction chamber plate 20 or may be integrally formed with the reaction chamber plate 20. The upper cover 10 includes a first blood injection port 11 and a second blood injection port 12 which are provided at predetermined positions adjacent to the rotation center such that the first blood injection port 11 and the second blood injection port 12 penetrate the cover, and a plurality of reagent injection ports 13 which are formed to be distributed in an arc shape at the edge side such that the plurality of reagent injection ports 13 penetrate the cover.

The first blood injection port 11, as a hole having the blood sample (whole blood) injected thereinto to supply blood sample (whole blood) to the blood receipt chamber 110 of the reaction chamber plate 20, is formed in a position corresponding to the blood receipt chamber 110 when the upper cover 10 is combined with the reaction chamber plate 20.

Further, the second blood injection port 12, as a hole having the blood sample (whole blood) injected thereinto to supply blood sample (whole blood) to the dyeing chamber 220 of the reaction chamber plate 20, is formed in a position corresponding to the dyeing chamber 220 when the upper cover 10 is combined with the reaction chamber plate 20.

Further, the plurality of reagent injection ports 13, as holes having the reagent injected thereinto to supply the reagent to the plurality of reagent reaction chambers 140 of the reaction chamber plate 20, are each formed in positions corresponding to the respective reagent reaction chambers 140 when the upper cover 10 is combined with the reaction chamber plate 20.

The reaction chamber plate 20, a disc container type plate which has a rotation center C and a predetermined depth dimension, and of which a top portion is opened, includes a biochemical examination unit 100 which measures concentration values of biochemical components of serum to diagnose vital functions, a blood testing unit 200 which separates blood samples to measure and test the number and deformation of red blood cells or white blood cells, and an immunity test unit 300 which measures an antigen-antibody reaction of serum to diagnose and test cancer or viruses, wherein the reaction chamber plate 20 is formed by dividedly forming the biochemical examination unit 100, blood testing unit 200 and immunity test unit 300 in a fan shape based on the rotation center C, and the reaction chamber plate 20 is detachably coupled to a lower portion of the upper cover 10 or integrally formed with the upper cover 10.

At this time, the reaction chamber plate 20 may include the biochemical examination unit 100 and the blood testing unit 200 which are only dividedly formed in a fan shape based on the rotation center C as illustrated in FIG. 4.

The biochemical examination unit 100, as a configuration for performing a biochemical examination of measuring an absorbance value to calculate a concentration value after injecting a blood sample (whole blood) into the biochemical examination unit 100, filtering a serum component only in the blood, and reacting the filtered serum component with each of reagents to perform a reaction process, may include a blood receipt chamber 110, a blood filter chamber 120, serum distribution chambers 130, and reagent reaction chambers 140.

A biochemical examination process comprises measuring an absorbance value when a chromogenic reaction occurs by reacting chemical components within serum with a test reagent for about 10 minutes, and converting the measured absorbance value into a concentration value using the Beer-Lambert Law to diagnose the vital functions by comparing vital functions (liver function, cardiac function, kidney function, etc.) with normal values.

The blood receipt chamber 110 is formed at a predetermined position adjacent to the rotation center C, and is formed at a position corresponding to the first blood injection port 11 of the upper cover 10 to receive a blood sample supplied through the injection port. Here, the blood receipt chamber 110 is preferably formed in an arc shape based around a rotation axis of the rotation center, and is formed to be communicated with the blood filter chamber 120 through a flow path.

The blood filter chamber 120, as a configuration for separating necessary blood components by filtering a transferred blood sample, is formed in an arc shape around the rotation axis of the rotation center, wherein a filter is provided within the chamber to separate serum only from the blood. An inlet side of the blood filter chamber 120 is communicated with the blood receipt chamber 110 through the flow path, and an outlet side of the blood filter chamber 120 is formed to be connected to a distribution flow path formed in an arc shape at an outer side of the rotation center.

At this time, the flow path connecting the blood receipt chamber 110 and the blood filter chamber 120 is preferably formed in an outermost line from the rotation center of each chamber to facilitate transfer of blood by rotational centrifugal force.

The serum distribution chambers 130, as a plurality of chambers to which a predetermined amount of serum, i.e., blood components filtered from the blood filter chamber 120 is distributed, is formed so that an inlet of each of the serum distribution chambers 130 is opened to an arc-shaped distribution flow path communicated with an outlet side of the blood filter chamber 120, and an outlet of each of the serum distribution chambers 130 is communicated with each of corresponding reagent reaction chambers 140 through a flow path.

In the reagent reaction chambers 140 as chambers for causing a reaction between supplied blood components and a reagent, a plurality of chambers are formed to be distributed in an arc shape, and formed in edge side positions corresponding to the reagent injection ports 13 of the upper cover 10 to receive serum, i.e., filtered blood components transferred from the serum distribution chambers 130 and receive reagents injected through the reagent injection ports 13. At this time, after dispensing dry-type indicators fitting to test items to be measured and drying the dispensed dry-type indicators, the dried dry-type indicators may be fixed to the reagent reaction chambers 140.

At this time, a connection flow path of the serum distribution chambers 130 and the reagent reaction chambers 140 is preferably formed to form a predetermined angle with respect to a rotational direction to facilitate transfer of blood by rotational centrifugal force. The blood components of the serum distribution chambers 130 are easily transferred to the reagent reaction chambers 140 during rotation of the multi-disc 1 by such a structure.

At this time, when the multi-disc 1 is rotated to a predetermined rotational speed (rpm) for a predetermined time, a blood sample supplied to the blood receipt chamber 110 is transferred to the blood filter chamber 120 by centrifugal force, blood components filtered in the blood filter chamber 120 are distributed to the serum distribution chambers 130 by centrifugal force, and the blood components are transferred from each of the serum distribution chambers 130 to each of the reagent reaction chambers 140.

The blood testing unit 200, as a configuration for separating blood samples to measure and test the number and deformation extent of red blood cells or white blood cells, may include a capillary tube holding chamber 210 and a dyeing chamber 220.

A blood test, as a test which has been most widely used in diagnostic test for the purpose of obtaining information on an overall health condition of a patient, indicates that there is a disease when the number of respective cells of white blood cells, red blood cells, platelet, and others circulated along a blood flow is abnormally high or low.

The capillary tube holding chamber 210 is for holding capillary tubes (not illustrated) containing blood samples, wherein the held capillary tubes containing blood samples are centrifuged by centrifugal force of a rotating multi-disc 1 such that the blood samples are fractionated into red blood cells (RBC), white blood cells (WBC), platelet and serum from bottom portions of the tubes.

At this time, when the multi-disc 1 is rotated at a rotation speed of 4,000 rpm for 10 minutes or rotated at a rotation speed of 10,000 rpm for 5 minutes, respective components within the blood samples are fractionated and divided by centrifugal force.

Blood cell counts of red blood cells, white blood cells, platelets, etc. are calculated by capturing separated images to measure length for each section, thereby calculating the total percent in a volume amount. Usually, there are approximately 45% of red blood cells, approximately less than 1% of white blood cells and platelets, and approximately 55% of plasma based on 100% of a normal blood sample.

Although the capillary tube holding chamber 210 is divided into five chambers, the number of the chambers is not limited to fiver, but the number of the chambers can be changed according to dimensions of capillary tubes held.

The dyeing chamber 220, as a chamber for discriminating and counting the white blood cells from dyed captured images by treating supplied blood samples with a dyeing reagent, thereby dyeing five types of white blood cells, is formed in a position corresponding to the second blood injection port 12 of the upper cover 10 to receive a blood sample (whole blood) injected through the second blood injection port 12. A dyeing solution may be provided within the chamber or may be received through the second blood injection port 12.

Usually, fiver types of white blood cells are Lymphocyte, Monocyte, Neutrophil, Eosinophil and Basophil, and a dyeing operation is carried out by mainly using Giemsa stain as a dyeing method. It goes without saying that the dyeing method is not limited to Giemsa stain.

A Giemsa solution includes AzureII-Eosin and Azure II as pigments, and the dyeing operation is conducted using a Giemsa solution obtained by fixing the pigments with methanol and diluting the fixed pigments with a phosphoric acid buffer solution or using a Wright-Giemsa solution making it not necessary to fix the pigments with methanol.

When carrying out the dyeing operation using the Giemsa solution, it is usual that the red blood cells have a pink color, the platelets have a light pink color, lymphocyte cytoplasm has a sky-blue color, monocyte cytoplasm has a light blue color, and white blood cell nuclear chromatin has a magenta color.

The immunity test unit 300, as a chamber for measuring an antigen-antibody reaction of serum to diagnose and test cancer or viruses, conducts an immunity testing operation of labeling and posting an indicator on a DNA fragment or an antibody (antigen) to be tested, fixing the DNA fragment or antibody (antigen) having the indicator labeled and posted thereon to a bottom surface, and measuring an amount of the antibody (antigen).

The immunity test unit 300 may include a structure (not illustrated) which centrifuges a blood sample injected through a separate injection port, and to which only a separated serum is supplied, or a structure which is communicated with the distribution flow path provided in the biochemical examination unit 100 to receive serum only, i.e., filtered blood components.

A multi-disc 1 according to the present invention may facilitate image capture or absorbance measurement after a reaction through the camera unit 3 by forming both of the upper cover 10 and the reaction chamber plate 20 of a transparent material.

The lighting unit 2 may be comprised of a light source 2a and a light filter 2b, and allows a required monochromatic light to be irradiated onto the multi-disc 1 from the light source through the light filter such as monochrometer.

The camera unit 3, as a high-resolution CCD camera or CMOS camera, is disposed above the multi-disc 1 to transmit the measured absorbance data or captured image data to the display unit 4 by measuring absorbance values after a reaction of the multi-disc 1 or capturing images.

At this time, an absorbance measurement unit such as a spectrophotometer for absorbance measurement may be additionally included.

A concentration value of a serum component may be calculated using the measured absorbance value at which a chromogenic reaction is exhibited, and the captured image data enable calculation of blood cell contents, discrimination and counting of white blood cells, and others.

At this time, the camera unit may enable an accurate measurement operation to be carried out with respect to an object having an ultra-fine structure or an object combined with a fluorescent dye by additionally including am optical and/or a fluorescent lens.

The display unit 4 is a device which displays the absorbance data and captured image data received from the camera unit 3, and the DB unit 5 is a device which is interlocked with the camera unit 3 and the display unit 4, stores the measured data, stores data compared with stored reference data, and provides the stored data to the display unit 4.

A multisystem for simultaneously performing a biochemical examination, a blood test and an immunity test configured as described above enables the biochemical examination and the blood test, or the biochemical examination, the blood test and the immunity test to be selectively performed at the same time.

Although the embodiments of the present invention are described with reference to the illustrated drawings, the above-disclosed subject matter is to be considered illustrative, and not restrictive, and the appended claims are intended to cover all such modifications, enhancements, and other embodiments, which fall within the true spirit and scope of the present invention. Thus, to the maximum extent allowed by law, the scope of the present invention is to be determined by the broadest permissible interpretation of the following claims and their equivalents, and shall not be restricted or limited by the foregoing detailed description.

## Claims

1. A multisystem for simultaneously performing a biochemical examination and a blood test, the multisystem including a multi-disc (1) in which a biochemical examination unit (100) and a blood testing unit (200) are divided and formed in a fan shape based on a rotation center.

2. The multisystem of claim 1, wherein the multi-disc (1) additionally includes an immunity test unit (300) to perform immunity test at the same time.

3. The multisystem of claim 1, wherein the multi-disc (1) includes an upper cover (10), and a reaction chamber plate (20) which is detachably combined with or integrally formed with the upper cover (10).

4. The multisystem of claim 3, wherein the upper cover (10) of the multi-disc (1) includes a first blood injection port (11) and a second blood injection port (12) which are provided at positions adjacent to the rotation center, and a plurality of reagent injection ports (13) which are formed to be distributed in an arc shape at the edge side, and
the reaction chamber plate (20) includes a biochemical examination unit (100) for measuring concentration values of biochemical components of serum to diagnose vital functions, a blood testing unit (200) for separating blood samples to measure and test the number and deformation of red blood cells or white blood cells, and an immunity test unit (300) for measuring an antigen-antibody reaction of serum to diagnose and test cancer or viruses.

5. The multisystem of claim 1, wherein the biochemical examination unit (100) of the multi-disc (1) includes:
a blood receipt chamber (110);
a blood filter chamber (120) which is provided to be communicated with the blood receipt chamber (110);
a plurality of serum distribution chambers (130) which are provided to be communicated with the blood filter chamber (120) through an arc-shaped distribution flow path; and
a plurality of reagent reaction chambers (140) which are provided to be communicated with the plurality of serum distribution chambers (130).

6. The multisystem of claim 5, wherein the blood receipt chamber (110) is formed in an arc shape around a rotation axis at a position adjacent to the rotation center to receive a blood sample supplied,
the blood filter chamber (120) is formed in an arc shape around the rotation axis of the rotation center and has a filter provided therein to filter a blood sample transferred,
the plurality of serum distribution chambers (130) is formed so that an inlet of each of the plurality of serum distribution chambers (130) is opened to the arc-shaped distribution flow path such that a predetermined amount of filtered blood components is distributed to each of the serum distribution chambers (130), and the inlet of each of the serum distribution chambers (130) is communicated with each of the corresponding reagent reaction chambers (140) through the flow path, and
the plurality of reagent reaction chambers (140) are formed to be distributed in the edge side, and the plurality of chambers are provided to cause a reaction between a reagent and the blood components supplied from the serum distribution chambers (130).

7. The multisystem of claim 5, wherein a flow path connecting the blood receipt chamber (110) to the blood filter chamber (120) is formed in an outermost line from the rotation center of each chamber to facilitate transfer of blood by rotational centrifugal force.

8. The multisystem of claim 5, wherein a connection flow path of the serum distribution chambers (130) and the reagent reaction chambers (140) is formed to form a predetermined angle with respect to a rotational direction to facilitate transfer of blood by rotational centrifugal force.

9. The multisystem of claim 1, wherein the blood testing unit (200) includes:
a capillary tube holding chamber (210) which holds tubes containing blood samples; and
a dyeing chamber (220) which is provided to discriminate and count the white blood cells by treating the blood samples with a dyeing reagent, thereby dyeing five types of white blood cells.

10. The multisystem of claim 2, wherein the immunity test unit (300) includes a structure which centrifuges a blood sample injected through a separate injection port, and to which only a separated serum is supplied, or a structure which is communicated with the distribution flow path provided in the biochemical examination unit (100) to receive filtered blood serum only.

11. The multisystem of claim 1, additionally including:
a lighting unit (2) which irradiates light onto the multi-disc (1);
a camera unit (3) which photographs the multi-disc (1);
a display unit (4) which displays data received from the camera unit (3); and
a DB unit (5).

12. The multisystem of claim 11, wherein the lighting unit (2) is comprised of a light source (2a) and a light filter (2b) to irradiate a monochromatic light onto the multi-disc (1), the camera unit (3) is disposed above the multi-disc (1) to transmit the measured absorbance data or captured image data to the display unit (4) by measuring absorbance values after a reaction of the multi-disc (1) or capturing images, the display unit (4) displays the absorbance data and image data received from the camera unit (3), and the DB unit (5) is interlocked with the camera unit (3) and the display unit (4), stores the measured data, and stores data compared with stored reference data.

13. A multi-disc including:
a biochemical examination unit (100) which measures concentration values of biochemical components of serum to diagnose vital functions;
a blood testing unit (200) which separates blood samples to measure and test the number and deformation of red blood cells or white blood cells; and
an immunity test unit (300) which measures an antigen-antibody reaction of serum to diagnose and test cancer or viruses, wherein the multi-disc is formed by dividedly forming the biochemical examination unit (100), blood testing unit (200) and immunity test unit (300) in a fan shape based on the rotation center.

14. The multi-disc of claim 13, wherein the multi-disc includes:
an upper cover (10) including a first blood injection port (11) and a second blood injection port (12) which are provided at positions adjacent to the rotation center, and a plurality of reagent injection ports (13) which are formed to be distributed in an arc shape at the edge side; and
a reaction chamber plate (20) which is coupled to the upper cover (10), and includes a biochemical examination unit (100) for measuring concentration values of biochemical components of serum to diagnose vital functions, a blood testing unit (200) for separating blood samples to measure and test the number and deformation of red blood cells or white blood cells, and an immunity test unit (300) for measuring an antigen-antibody reaction of serum to diagnose and test cancer or viruses.

15. The multi-disc of claim 14, wherein the biochemical examination unit (100) of the multi-disc includes: a blood receipt chamber (110) which is provided at a position corresponding to the first blood injection port (11); a blood filter chamber (120) which is provided to be communicated with the blood receipt chamber (110); a plurality of serum distribution chambers (130) which are provided to be communicated with the blood filter chamber (120) through an arc-shaped distribution flow path; and a plurality of reagent reaction chambers (140) which are provided to be communicated with the plurality of serum distribution chambers (130) and provided at positions corresponding to the reagent injection ports (13),
the blood testing unit (200) of the multi-disc includes: a capillary tube holding chamber (210) which holds tubes containing blood samples; and a dyeing chamber (220) which is provided to discriminate and count the white blood cells by treating the blood samples with a dyeing reagent, thereby dyeing five types of white blood cells, and
the immunity test unit (300) of the multi-disc includes a structure which centrifuges a blood sample injected through a separate injection port, and to which only a separated serum is supplied, or a structure which is communicated with the distribution flow path provided in the biochemical examination unit (100) to receive filtered blood serum only.
